# EUROPEAN PATENT APPLICATION

(11) **EP 4 342 879 A1**
(43) Date of publication of application: **27.03.2024**
(21) Application number: 22196808.4
(22) Date of filing: 21.09.2022
(51) Int. Cl.: C07C 213/02, C07C 231/02, C07C 233/22, C07C 217/60

(54) **VERY EFFICIENT PROCESS FOR THE PREPARATION OF AGOMELATINE**

(71) Applicant: F.I.S.- Fabbrica Italiana Sintetici S.p.A., 36075 Montecchio Maggiore (VI) (IT)
(72) Inventor: Defilippo, Christian, 36075 Montecchio Maggiore (IT); Motterle, Riccardo, 36075 Montecchio Maggiore (IT)
(74) Representative: Leganza, Alessandro

(57) **Abstract**

The present invention is directed to a very efficient process for the preparation of Agomelatine wherein the intermediate 7-methoxynaphtalenacetonitrile is reduced by Lithium Aluminium hydride in presence of a Lewis acid.

## Description

### Technical field

The present invention is related to a synthetic process for the preparation of a small molecule, being an active pharmaceutical ingredient named Agomelatine.

### State of the Art

Agomelatine is an active pharmaceutical ingredient of formula (I): 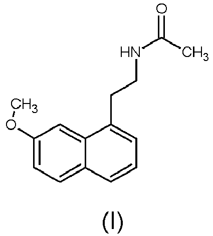 having IUPAC name N-[2-(7-methoxynaphthalen-1-yl)ethyl]acetamide and used for the treatment of major depressive disorders.

Agomelatine can be prepared according to several routes of synthesis whose an efficient one is the route of synthesis that foresees the reduction reaction of the intermediate named 7-methoxynaphtalenacetonitrile of formula (III): 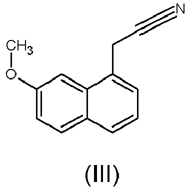 to give 2-(7-methoxynaphthalen-1-yl)ethan-1-amine of formula (II) or salts thereof:

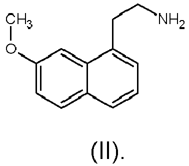

The intermediate of 2-(7-methoxynaphthalen-1-yl)ethan-1-amine of formula (II) or salts thereof can easily be converted to Agomelatine of formula (I) or salts, solvates or co-crystals thereof, by means of an acetylation reaction, for instance by reaction with acetic anhydride or acetylchloride.

The intermediate 7-methoxynaphtalenacetonitrile of formula (III) can be easily prepared by dehydration reaction of the corresponding amide, i.e. by dehydration reaction of of 2-(7-methoxynaphthalen-1-yl)acetamide, for instance by means so thionyl chloride in dimethylformamide solvent.

Finally, the thus produced Agomelatine can be further converted into an Agomelatine co-crystal, e.g. can be converted into Agomelatine-Urea co-crystal (1:1) by mixing Agomelatine and urea, preferably in a solvent medium.

A crucial step of the above mentioned route of synthesis is the 7-methoxynapthaleneacetonitrile reduction to generate the primary amine, i.e. to give 2-(7-methoxynaphthalen-1-yl)ethan-1-amine of formula (II) or salts thereof, in particular to give 2-(7-methoxynaphthalen-1-yl)ethan-1-amine hydrochloride.

The publication by Keith Smith, Gamal A. El-Hiti, Mohammed B. Alshammari, SYNTHESIS 2014, 46, 0394-0402, disclosed the reduction reaction of 7-methoxynapthaleneacetonitrile carried out in methanol medium in a presence of hydrochloric acid and Pd/C as catalyst. (see scheme 1 below):

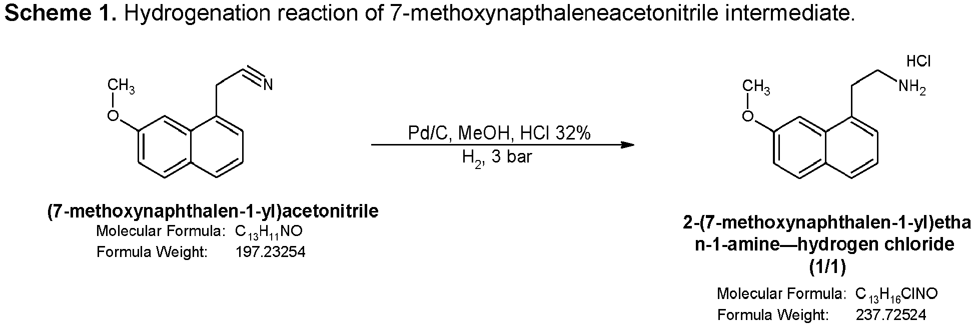

The reaction is performed in a high dilution condition near 20 volumes of solvent and required higher loading of catalyst (in the range 0.1-0.2 W respect to starting nitrile) and required a significant reaction time up to 48 hrs. These reaction conditions significantly impact on the amount of waste generated by the process and the full cost of the final Agomelatine, mainly because of the high cost of Palladium catalyst.

Considering the literature, many approaches to the reduction of nitrile moiety can be applied to substitute the Pd/C and reduce the amount of solvent used in the hydrogenation reaction. The first tentative reported in the patent document EP1564202A1 described a reduction of 7-methoxynapthaleneacetonitrile in a presence of ammonia using Nichel-Raney as catalyst (see scheme 2 below) 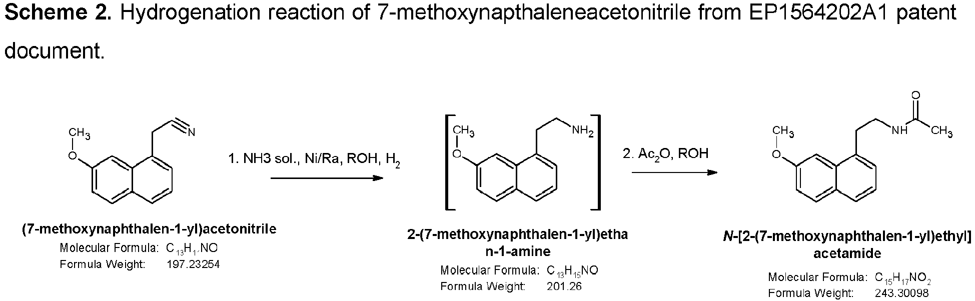 Note: ROH is methanol or Ethanol.

The reaction was tested in Lab. by focusing on the first stage of the process by performing the reaction in a presence and absence of acetic anhydride following the reaction conditions reported in said patent document (see experimental section for the details). The reaction conversion is reported below in the following table 1.

**Table 1. Ni/Ra basic conditions, reaction conversion.**

| ID experiment | Presence of Ac₂O | reaction conversion HPLC %A |
|---|---|---|
| RD-016-2718-2642 | NO | 99.95 |
| RD-016-2718-2643 | YES | 100 |

In both cases the reaction conversion appears as quantitative and two different materials were formed: the free amine in the experiment carried out in absence of acetic anhydride and the acetyl amine in the reaction performed in a presence of Ac₂O. However, a significant amount of by-product was observed at the end of reaction (HPLC chromatograms shows peaks at retention time longer than 2-(7-methoxynaphthalen-1-yl)ethan-1-amine and Agomelatine).

The isolated material shows a low purging factor of these impurities for both experiments, as show in the table 2 below

**Table 2. HPLC lot analysis of isolated material.**

| ID experiment | HPLC purity %A | RRT 1.028 | RRT 1.395 | RRT 2.095 | RRT 1.57 |
|---|---|---|---|---|---|
| RD-016-2718-2642 | 98.41 | N.D. | N.D. | 1.57 | N.D. |
| RD-016-2718-2643 | 93.32 | 2.25 | 0.25 | N.D. | 3.95 |
| SPECIFICATIONS | NLT 99.0 | NMT 0.10 | NMT 0.10 | NMT 0.10 | NMT 0.10 |

| | | | | | |
|---|---|---|---|---|---|
| Note: N.D. is not detected | | | | | |

Further investigation performed using an ESI-MS techniques, suggest as the unknow impurities detected in the IPC and in the lot analysis can be the results of the reaction between the in situ generated free-amine with the unreacted nitrile starting material. The immine product can be reduced by Ni/Ra at secondary amine which is able to react another time with nitrile raw material to generate tertiary amine or a trimeric amine as described in the reaction scheme 3 below.

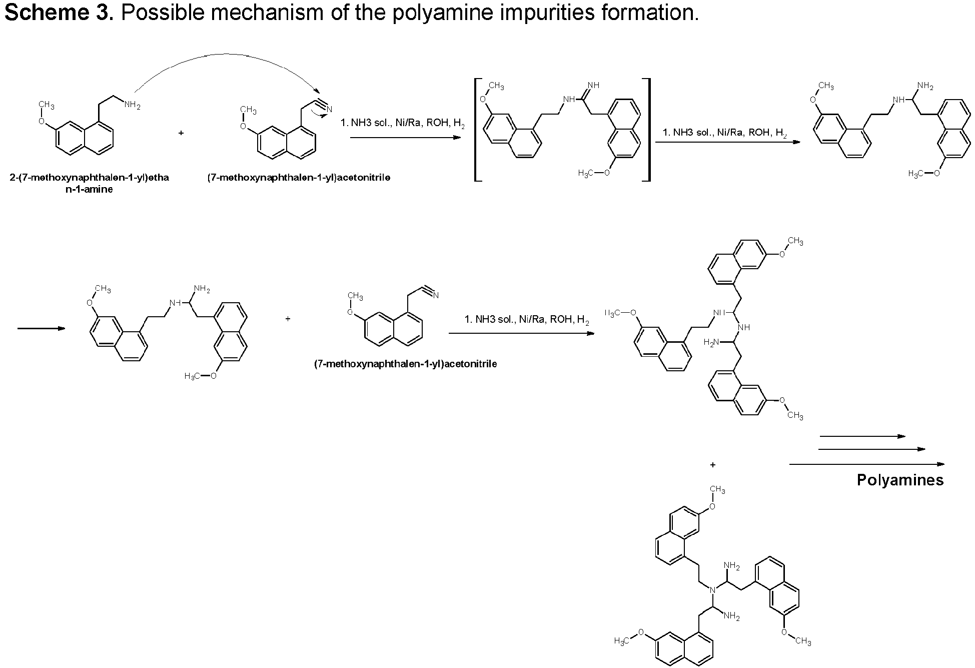

In the method disclosed in literature where the reduction of nitrile group is performed with Pd/C, the polymerization of amine is inhibited by addition in the reaction mass of hydrochloric acid, the free amine generated by the hydrogenation reaction is immediately quenched by acid-base reaction with HCI. Several experiment with Ni/Ra in acid medium was performed but very low reaction conversion was observed, the acid environment is able to hydrolyze the catalyst and compromised the catalytic efficiency of the Nichel Raney and poisoning the products with Nichel. Considering the chemical properties of the nitrile and based on the previous results obtained in the first experiments, a different reduction approach was needed to overcome the outstanding problems with reference to the processes belonging to the prior art.

### Detailed description of the invention

Thus, an object of the present invention is a process for the preparation of Agomelatine of formula (I) or salts, solvates or co-crystals thereof: 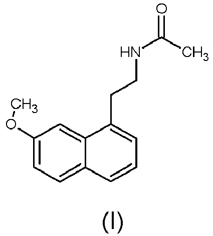 comprising the following steps:
a) reduction reaction of 7-methoxynaphtalenacetonitrile of formula (III): 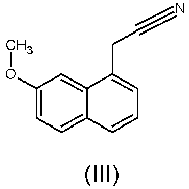 to give 2-(7-methoxynaphthalen-1-yl)ethan-1-amine of formula (II) or salts thereof: 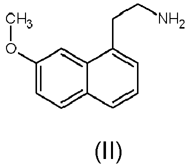
b) conversion of 2-(7-methoxynaphthalen-1-yl)ethan-1-amine of formula (II) or salts thereof of step a) to Agomelatine of formula (I) or salts, solvates or co-crystals thereof, by means of acetylation reaction,
   **characterized in that** the reduction reaction of the step a) is performed with LiAlH₄ in presence of a Lewis acid.

Indeed, it has surprisingly found that the presence of a Lewis acid during the reduction reaction with Lithium Aluminum hydride (LiAlH₄) allows to react extraordinary results both in terms of molar yield and, especially, in chemical purity of product obtained as outlined in the following description.

The new reduction agent was evaluated as alternative reagent in the nitrile reduction instead of Pd/C or Nichel Raney catalyst.

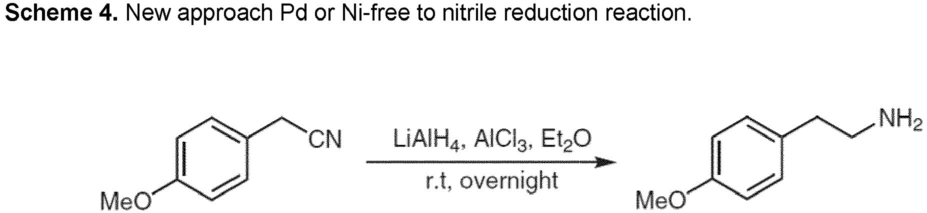

The use of a Lewis acid, e.g. an aluminium salt can complex the amide generated by LiAlH₄ to avoid the polymerization and increase the electrophilicity of the nitrile moiety as described in the scheme 5 below.

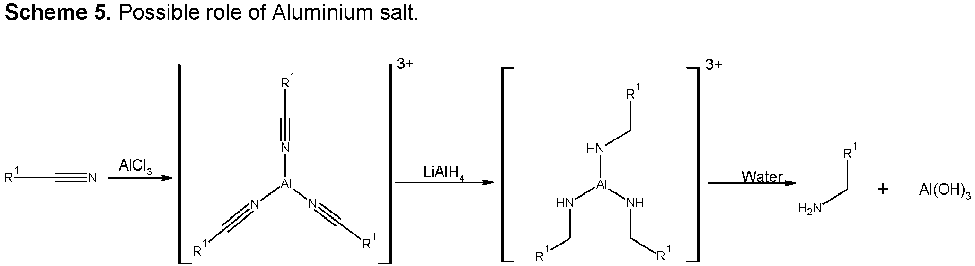

To verify this hypothesis and confirm the role of aluminium trichloride as Lewis acid in the system, an experiment was carried out by using only LiAlH₄ as reagent without charge AlCl₃. (see table 3 below)

**Table 3. Reaction conversion of nitrile reduction without AlCl₃ loading.**

| ID experiment | HPLC conv % | HPLC %A product | Total organic impurities %A | Major unk RRT | Major unk %A |
|---|---|---|---|---|---|
| RD-016-2718-2690 | 40 | 46.7 | 21.79 | 1.84 | 8.27 |

A large amount of organic by-product was observed, furthermore a non complete reaction conversion was observed however, in a presence of AlCl₃ the reaction conversion was complete after 1-1,5 hrs as reaction time with a quantitative selectivity (see table 4 below).

**Table 4. Role of AlCl₃.**

| ID experiment | eq. AlCl₃ | eq. LiAIH 4 | Solvent | HPLC conv% | HPLC %A product | Total organic impurities %A | Major unk. RRT | Major unk. %A |
|---|---|---|---|---|---|---|---|---|
| RD-016-2742-2699 | 0.36 5 | 1.1 | Toluen e/THF | 100 | 99.92 | 0.07 | 1.68 | 0.07 |
| RD-016-2718-2690 | 0 | 1.1 | Toluen e/THF | 40 | 46.7 | 21.79 | 1.84 | 1.84 |

The product can be isolated as acetyl amine derivative or HCI salt using HCI in methyl tertbutyl ether, as reported in the scheme below.

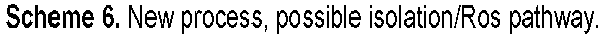

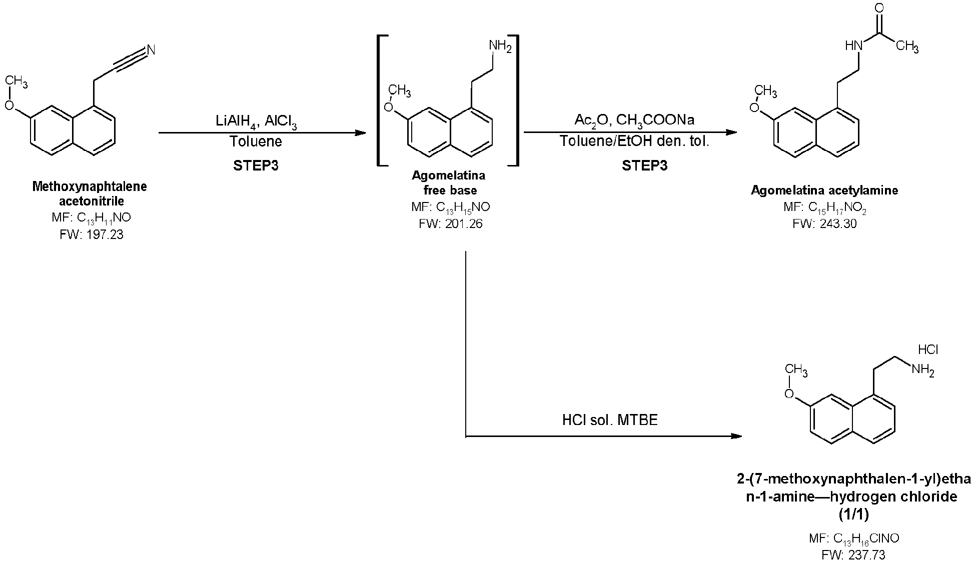

Both approaches led a high-quality intermediate, with a good isolated yield as reported in the table 5 below.

**Table 5. New method development, results of isolation methods**

| ID experim ent | eq. AlCl₃ | eq. LiAlH₄ | Final product isolated | HPLC conv% (reducti on reactio n) | HPLC conv% acetylat ion | Isolate d yeld | HPLC purity %A | Total organic impuriti es %A | Major unk. RRT | Major unk. %A |
|---|---|---|---|---|---|---|---|---|---|---|
| RD-016-2742-2699 | 0.365 | 1.1 | Acetyl amine | 100 | 99.94 | 88.9 | 100 | / | / | / |
| RD-016-2718-2689 | 0.365 | 1.1 | HCI salt | 100 | / | 87.5 | 100 | / | / | / |

The RPG factor of the synthesis of Agomelatine, only by substitute the hydrogenation step with the reduction of LiAlH₄/AlCl₃ (isolation of HCI salt), goes up to 70 % and no heavy metal waste was generated, in a face of a reduction of raw materials cost of 57%.

The molar yield of 2-step Agomelatine synthesis is thus from 87% to 89% and the product shows an HPLC purity of 100.00% (HPLC A/A%).

The process of synthesis of Agomelatine based on the new method of reduction of 7-methoxynaphthalenacetonitrile shows a very high selectivity and flexibility, that decrease the raw material cost of the Agomelatine synthesis and allows to decrease the amount of waste generated per kilograms of API synthetized.

Due to the structure of the process is possible to isolate the 2-(7-methoxynaphthalen-1-yl)ethan-1-amine of formula (II) hydrocholoride or the Agomelatine in good yield with quantities HPLC purity.

According to one aspect of the process, in the reduction reaction, the Lewis acid can be selected among AlCl₃, AlF₃ and BF₃. Preferably, the Lewis acid is AlCl₃.

According to another aspect of the process, in the reduction reaction, the Lewis acid is used in an amount from 0.33 to 0.40 or from 0.355 to 0.375 molecular equivalents compared to 7-methoxynaphtalen acetonitrile of formula (III).

According to another aspect of the process, in the reduction reaction, according to any one of the claims from 1 to 4, wherein the LiAlH₄ is used in an amount from 1.0 to 1.20 or from 1.05 to 1.15 molecular equivalents compared to 7-methoxynaphtalenacetonitrile of formula (III).

According to another aspect of the process, the reduction reaction of step a) is carried out in Toluene.

According to another aspect of the process, the reduction reaction of step a) is carried out by mixing LiAlH₄ and the Lewis acid and then by adding 7-methoxynaphtalenacetonitrile of formula (III).

According to another aspect of the process, the reduction reaction of step a) is carried out at a temperature comprised in the range from 0°C to 25°C.

According to a preferred aspect of the process, the salt of 2-(7-methoxynaphthalen-1-yl)ethan-1-amine of formula (II) is the hydrochloride salt.

According to a preferred aspect of the process, the Agomelatine co-crystal is Agomelatine-Urea co-crystal (1:1).

Another object is a process for the preparation of 2-(7-methoxynaphthalen-1-yl)ethan-1-amine of formula (II) or salts thereof: 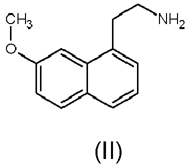 by means of reduction reaction of 7-methoxynaphtalenacetonitrile of formula (III): 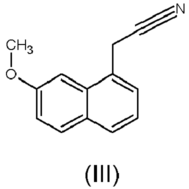 wherein said reduction reaction is performed with LiAlH₄ in presence of a Lewis acid.

According to a preferred embodiment, the reduction reaction is carried out according the condition of above described with reference of the above said process for the preparation of Agomelatine.

### Experimental section

### Example 1: Process for the preparation of Agomelatine (RD-016-2742-2699)

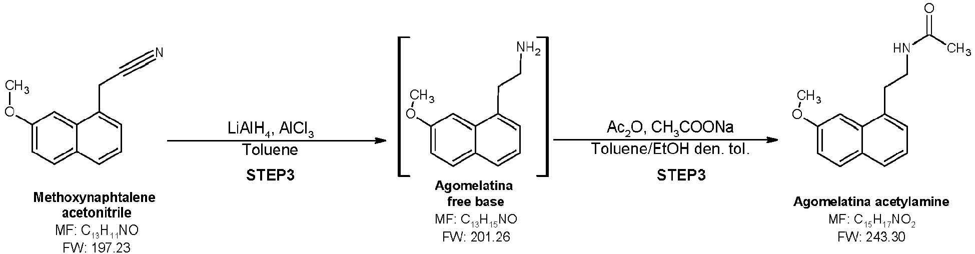

In a glass made reactor under nitrogen atmosphere 120 mL (3.0 V) of dry toluene was charged at T =5-10 °C. On the solvent 9.9 g (0.074 mol., 0.365 eq.) of anhydrous Aluminum trichloride. The charging line was washed with 10 mL (0.25 V) of dry toluene. On the resulting suspension, 108 mL (0.216 mol., 1.1 eq.) of 2.0 M LiAlH₄ THF solution were added dropwise on the suspension keeping the internal temperature below T=25 °C with a typical addition time of about 30 minutes. At the end of additions, the charging line was washed with 10 mL (0.25 V) of dry toluene. On the light green-yellow mixture a solution of 40 g (0.2028 mol., 1.0 eq.) of 7-mothoxynapthalene acetonitrile dissolved in 160 mL (4.0 V) of dry toluene were added in a period of 60 minutes keeping the internal temperature below T=25 °C. At the end of addition, the charging line was washed with 10 mL (0.25 V) of dry toluene. The reaction mixture was heated and kept at T=22/25 °C for 30 minutes. A sample of the reaction mixture was taken, IPC A010 conv %.

In a second reactor, 200 mL (5.0 V) of toluene and 120 mL (3.0 V) of purified water were charged at T=2/5 °C. The reaction mass was transferred in a period of 90 minutes on the mixture toluene/water keeping the temperature in the second reactor below T=20/25 °C. At the end of addition the charging line was washed with 10 mL (0.25 V) of toluene. The resulting white suspension was kept at T=0/5 °C for 30 minutes than filtered on paper filter. The panel was suspended with 200 mL (5.0 V) of toluene for 30 minutes at T=20/25 °C and filtered. The panel was washed with 80 mL (2.0 V) of toluene. the combined mixture was distilled at T_{jacket} =42/45 °C under vacuum up to residue. The oil residue was diluted at T=20/45 °C with 160 mL (4.0 V) of toluene and heated at T=42/45 °C. On the organic mixture, 80 mL (2.0 V) of purified water were charged at T=42/45 °C. The bi-phasic mixture was kept under stirring for 10 minutes at T=42/45 °C and led to settle for 10 minutes. The phases were separated at T=42/45 °C. On the organic phase at T=42/45 °C, 80 mL (2.0 V) of purified water were charged at T=42/45 °C. The bi-phasic mixture was kept under stirring for 10 minutes at T=42/45 °C and led to settle for 10 minutes than separated at T=42/45 °C. The organic phase was distilled at T_{jacket} =42/45 °C under vacuum up to residue. The oil was diluted at T=20/45 °C with 153 mL (3.83 V) of dry toluene and 42 mL (1.05 V) of Ethanol den. toluene. A sample of the reaction mixture was taken, IPC A020 KF 479 ppm. The resulting solution was cooled down at T=0/5 °C and 17.5 g (0.213 mol., 0.213 eq.) of anhydrous sodium acetate were added. The charging line was washed with 10 mL (0.25 V) of dry toluene. On the reaction suspension, 21.74 g (0.213 mol., 1.05 eq.) of acetic anhydride were added dropwise keeping the internal temperature below 10 °C. At the end of additions the charging line was washed with 10 mL (0.25 V) of dry toluene. The resulting solution was heated at T=20/25 °C and kept at this temperature for 30-60 minutes. A sample of the reaction mass was taken, IPC 030.

At the end of the reaction 120 mL (3.0 V) of purified water were slowly added, at the end of addition the reaction mass was heated at T=45/50 °C and kept at this temperature for 15 minutes. The phases were led to settle for 10 minutes and separated. The organic layer was wash at T=45/50 °C with 80 mL (2.0 V) and 40 mL (1.0 V) respective of purified water. The resulting organic layer was distilled up to white solid residue under vacuum at T_{jacket} =45/50 °C. The solid is diluted at T=45/50 °C with 120 mL (3.0 V) of toluene and heated at T=73/75 °C to dissolve the solid. The solution was cooled down at T=0/-2 °C in a period of 2 hrs, the suspension was kept at T=0/-2 °C for 2hrs then filtered at T=0/-2 °C. The panel was washed with 40mL (1.0 V) of T=0/-2°C cold toluene. The solid was dried under vacuum at T=30/35 °C for 12 hrs to obtained 43,8 g (0.180 mol., 0.889 eq., 88.9 % mol. yield) of white solid.

### Example 2: Process for the preparation of Agomelatine (RD-016-2718-2689)

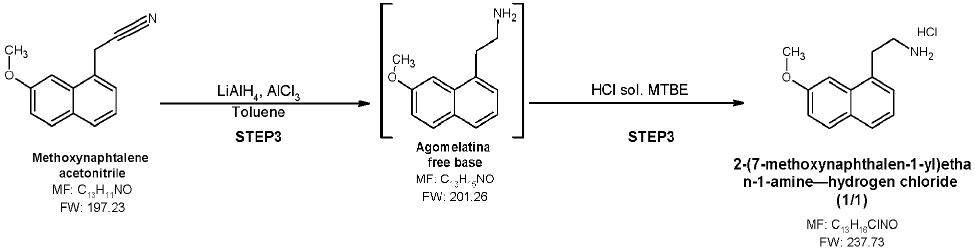

In a glass made reactor under nitrogen atmosphere 60 mL (3.0 V) of dry toluene was charged at T =5-10 °C. On the solvent 5.0 g (0.074 mol., 0.365 eq.) of anhydrous Aluminum trichloride. The charging line was washed with 5 mL (0.25 V) of dry toluene. On the resulting suspension, 54 mL (0.216 mol., 1.1 eq.) of 2.0 M LiAlH₄ THF solution were added dropwise on the suspension keeping the internal temperature below T=25 °C with a typical addition time of about 30 minutes. At the end of additions, the charging line was washed with 5 mL (0.25 V) of dry toluene. On the light green-yellow mixture a solution of 20 g (0.2028 mol., 1.0 eq.) of 7-mothoxynapthalene acetonitrile dissolved in 80 mL (4.0 V) of dry toluene were added in a period of 60 minutes keeping the internal temperature below T=25 °C. At the end of addition, the charging line was washed with 5 mL (0.25 V) of dry toluene. The reaction mixture was heated and kept at T=22/25 °C for 30 minutes. A sample of the reaction mixture was taken, IPC A010 conv %.

In a second reactor, 100 mL (5.0 V) of toluene and 60 mL (3.0 V) of purified water were charged at T=2/5 °C. The reaction mass was transferred in a period of 90 minutes on the mixture toluene/water keeping the temperature in the second reactor below T=20/25 °C. At the end of addition the charging line was washed with 5 mL (0.25 V) of toluene. The resulting white suspension was kept at T=0/5 °C for 30 minutes than filtered on paper filter. The panel was suspended with 100 mL (5.0 V) of toluene for 30 minutes at T=20/25 °C and filtered. The panel was washed with 40 mL (2.0 V) of toluene. the combined mixture was distilled at T_{jacket} =42/45 °C under vacuum up to residue. The oil residue was diluted at T=20/45 °C with 80 mL (4.0 V) of toluene and heated at T=42/45 °C. On the organic mixture, 40 mL (2.0 V) of purified water were charged at T=42/45 °C. The bi-phasic mixture was kept under stirring for 10 minutes at T=42/45 °C and led to settle for 10 minutes. The phases were separated at T=42/45 °C. On the organic phase at T=42/45 °C, 40 mL (2.0 V) of purified water were charged at T=42/45 °C. The bi-phasic mixture was kept under stirring for 10 minutes at T=42/45 °C and led to settle for 10 minutes, then separated at T=42/45 °C. The organic phase was distilled at T_{jacket} =42/45 °C under vacuum up to oily residue. The residue was diluted at T=20/45 °C with 60 mL (3.0 V) of Toluene and 40 mL (2.0 V) of Ethanol den. toluene. A sample of the reaction mixture was taken, IPC A020 KF (424 ppm). The solution was cooled down at T=0/10 °C and 35 mL (0.140 mol., 1.38 eq.) of 14,6 %w HCI solution in MTBE, keeping the internal temperature below T=10 °C. The charging line was washed with a T=0/10 °C pre-cooled solution of 30 mL (1.5 V) of toluene and 20 mL (1.0 V) of Ethanol den. toluene. The with suspension was kept at T=0/5 °C for 1 h than filtered. The panel was washed with 40 mL (2.0 V) of toluene and dried under vacuum at T=42/45 °C for 8 h to obtained 21.1 g (0.08876 mol., 87.5 % mol yield) of white solid.

## Claims

1. A process for the preparation of Agomelatine of formula (I) or salts, solvates or co-crystals thereof: 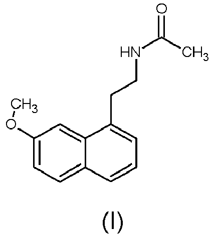 comprising the following steps:
c) reduction reaction of 7-methoxynaphtalenacetonitrile of formula (III): 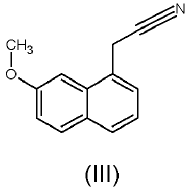 to give 2-(7-methoxynaphthalen-1-yl)ethan-1-amine of formula (II) or salts thereof: 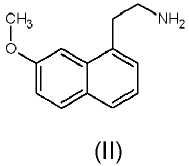
d) conversion of 2-(7-methoxynaphthalen-1-yl)ethan-1-amine of formula (II) or salts thereof of step a) to Agomelatine of formula (I) or salts, solvates or co-crystals thereof, by means of acetylation reaction,
**characterized in that** the reduction reaction of the step a) is performed with LiAlH₄ in presence of a Lewis acid.

2. Process according to claim 1, wherein the Lewis acid is selected among AlCl₃, AlF₃ and BF₃.

3. Process according to any one of the claims from 1 to 2, wherein the Lewis acid is AlCl₃.

4. Process according to any one of the claims from 1 to 3, wherein the Lewis acid is used in an amount from 0.33 to 0.40 or from 0.355 to 0.375 molecular equivalents compared to 7-methoxynaphtalen acetonitrile of formula (III).

5. Process according to any one of the claims from 1 to 4, wherein the LiAlH₄ is used in an amount from 1.0 to 1.20 or from 1.05 to 1.15 molecular equivalents compared to 7-methoxynaphtalenacetonitrile of formula (III).

6. Process according to any one of the claims from 1 to 5, wherein the reduction reaction of step a) is carried out in Toluene.

7. Process according to any one of the claims from 1 to 6, wherein the reduction reaction of step a) is carried out by mixing LiAlH₄ and the Lewis acid and then by adding 7-methoxynaphtalenacetonitrile of formula (III).

8. Process according to any one of the claims from 1 to 7, wherein reduction reaction of step a) is carried out at a temperature comprised in the range from 0°C to 25°C.

9. Process according to any one of the claims from 1 to 8, wherein the salt of 2-(7-methoxynaphthalen-1-yl)ethan-1-amine of formula (II) is the hydrochloride salt.

10. Process according to any one of the claims from 1 to 9, wherein the Agomelatine co-crystal is Agomelatine-Urea co-crystal (1:1).

11. Process for the preparation of 2-(7-methoxynaphthalen-1-yl)ethan-1-amine of formula (II) or salts thereof: 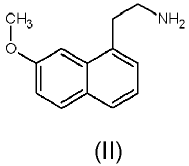 by means of reduction reaction of 7-methoxynaphtalenacetonitrile of formula (III): 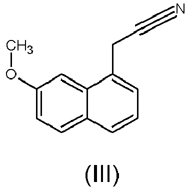 wherein said reduction reaction is performed with LiAlH₄ in presence of a Lewis acid.

12. Process according to claim 11, wherein the reduction reaction is carried out according the condition of any one of the claims from 2 to 9.
